# EUROPEAN PATENT SPECIFICATION

(11) **EP 2 060 266 B1**
(45) Date of publication and mention of the grant of the patent: **10.08.2011**
(21) Application number: 09075024.1
(22) Date of filing: 17.03.2005
(51) Int. Cl.: A61K 38/17, C07K 14/705, A61P 3/04, A61P 1/08

(54) **Y4 selective receptor agonist PP2-36 for therapeutic interventions**
Y4-selektiver Rezeptoragonist PP2-36 für therapeutische Eingriffe
PP2-36 un agoniste du récepteur de sélection Y4 pour interventions thérapeutiques

(30) Priority: 17.03.2004 DK 200400436; 01.04.2004 US 558932 P; 06.07.2004 US 585964 P; 14.02.2005 GB 0503110
(43) Date of publication of application: 20.05.2009
(62) Divisional of application: 05716258.8
(73) Proprietor: 7TM Pharma A/S, 2970 Horsholm (DK)
(72) Inventor: Schwartz, Thue, 2000 Frederiksberg (DK)
(74) Representative: Walls, Alan James

(56) References cited:
- WO-A-2005/077094
- US-A- 5 830 434
- US-A1- 2004 209 807
- BARD JONATHAN A ET AL: "Cloning and functional expression of a human Y4 subtype receptor for pancreatic polypeptide, neuropeptide Y, and peptide YY" JOURNAL OF BIOLOGICAL CHEMISTRY, vol. 270, no. 45, 1995, pages 26762-26765, XP002187958 ISSN: 0021-9258
- GEHLERT D R ET AL: "Characterization of the peptide binding requirements for the cloned human pancreatic polypeptide-preferring receptor" MOLECULAR PHARMACOLOGY 199607 US, vol. 50, no. 1, July 1996 (1996-07), pages 112-118, XP008104982 ISSN: 0026-895X
- BERNTSON G G ET AL: "Pancreatic polypeptide infusions reduce food intake in Prader-Willi syndrome" PEPTIDES 1993 US, vol. 14, no. 3, 1993, pages 497-503, XP002522820 ISSN: 0196-9781
- BATTERHAM R L ET AL: "Pancreatic polypeptide reduces appetite and food intake in humans" JOURNAL OF CLINICAL ENDOCRINOLOGY AND METABOLISM 20030801 US, vol. 88, no. 8, 1 August 2003 (2003-08-01), pages 3989-3992, XP002522821 ISSN: 0021-972X
- ASAKAWA A ET AL: "Characterization of the effects of pancreatic polypeptide in the regulation of energy balance" GASTROENTEROLOGY 20030501 US, vol. 124, no. 5, 1 May 2003 (2003-05-01), pages 1325-1336, XP002522822 ISSN: 0016-5085
- MCCREA KAREN ET AL: "2-36(K4,RYYSA19-23)PP a novel Y5-receptor preferring ligand with strong stimulatory effect on food intake" REGULATORY PEPTIDES, vol. 87, no. 1-3, 8 February 2000 (2000-02-08), pages 47-58, XP002352457 ISSN: 0167-0115
- CABRELE CHIARA ET AL: "Y-receptor affinity modulation by the design of pancreatic polypeptide/neuropeptide Y chimera led to Y5-receptor ligands with picomolar affinity" PEPTIDES, ELSEVIER, AMSTERDAM, vol. 22, no. 3, 1 March 2001 (2001-03-01), pages 365-378, XP002339076 ISSN: 0196-9781
- JOERGENSEN J C ET AL: "STRUCTURE-FUNCTION STUDIES ON NEUROPEPTIDE Y AND PANCREATIC POLYPEPTIDE-EVIDENCE FOR TWO PP-FOLD RECEPTORS IN VAS DEFERENS" EUROPEAN JOURNAL OF PHARMACOLOGY, AMSTERDAM, NL, vol. 186, no. 1, 4 September 1990 (1990-09-04), pages 105-114, XP002339197 ISSN: 0014-2999
- FUHLENDORFF J ET AL: "THE ANTIPARALLEL PANCREATIC POLYPEPTIDE FOLD IN THE BINDING OF NEUROPEPTIDE Y TO Y-1 AND Y-2 RECEPTORS" JOURNAL OF BIOLOGICAL CHEMISTRY, AMERICAN SOCIETY OF BIOLOCHEMICAL BIOLOGISTS, BIRMINGHAM,, US, vol. 265, no. 20, 15 July 1990 (1990-07-15), pages 11706-11712, XP002339077 ISSN: 0021-9258
- GINGERICH R L ET AL: "Patterns of immunoreactive pancreatic polypeptide in human plasma" REGULATORY PEPTIDES, ELSEVIER SCIENCE BV, NL, vol. 33, no. 3, 17 May 1991 (1991-05-17), pages 275-285, XP025208927 ISSN: 0167-0115 [retrieved on 1991-05-17]
- WALKER M W ET AL: "A STRUCTURE-ACTIVITY ANALYSIS OF THE CLONED RAT AND HUMAN Y4 RECEPTORS FOR PANCREATIC POLYPEPTIDE", PEPTIDES, ELSEVIER, AMSTERDAM, vol. 18, no. 4, 1 January 1997 (1997-01-01), pages 609-612, XP000876915, ISSN: 0196-9781, DOI: DOI:10.1016/S0196-9781(97)00070-3

## Description

### FIELD OF THE INVENTION

The invention relates to pancreatic peptide PP2.36 with SEQ. ID N.10 that acts as a selective agonist of the Y4 relative to the Y1 and Y2 receptors, and to its use in treatment of conditions responsive to activation of Y4 receptors, for example for treatment of obesity and overweight, and conditions in which these are considered contributory factors, diarrhoea and intestinal hypersecretion.

### BACKGROUND TO THE INVENTION

**The PP-fold family of peptides** - NPY (Neuropeptide Y) (human sequence - SEQ ID. No:1), PYY (Peptide YY) (human sequence-SEQ ID. No:2), and PP (Pancreatic Polypeptide) (human sequence - SEQ ID. No:3), are naturally secreted homologous, 36 amino acid, C-terminally amidated peptides, which are characterized by a common three-dimensional, structure - the PP-fold - which is surprisingly stable even in dilute aqueous solution and is important for the receptor recognition of the peptides.

Initially the X-ray structure of avian PP was characterized in great detail through X-ray crystallographic analysis down to a resolution of 0.98 A and the unique structure obtained its name from this peptide *(*Blundell et al. 1981 Proc.Natl.Acad.Sci.USA 78: 4175-79*;* Glover et al. 1984, Eur.J.Biochem. 142: 379-85*).* Subsequently, the PP-fold structure of other members of the family have been analysed through especially NMR spectroscopic analysis. Both X-ray and NMR analysis are obviously performed in very concentrated or solid conditions; however, detailed circular dichroism analysis suggests that NPY and PP even in aqueous solution adopt the PP-fold structure, which is unusual for such a small peptide (Fuhlendorff et al. 1990 J.BioLChem. 265: 11706-12). Importantly, analysis of the proteolytic stability of the peptides and fragments and analogs of these strongly indicate that for example the full length PP1-36 even in dilute aqueous solution is held in a folded configuration which protects it from degradation by certain enzymes which readily and rapidly degrade analogs which cannot adopt the PP-fold structure due to minor substitutions (Schwartz et al. 1990 Annals NY Acad. Sci. 611: 35-47).

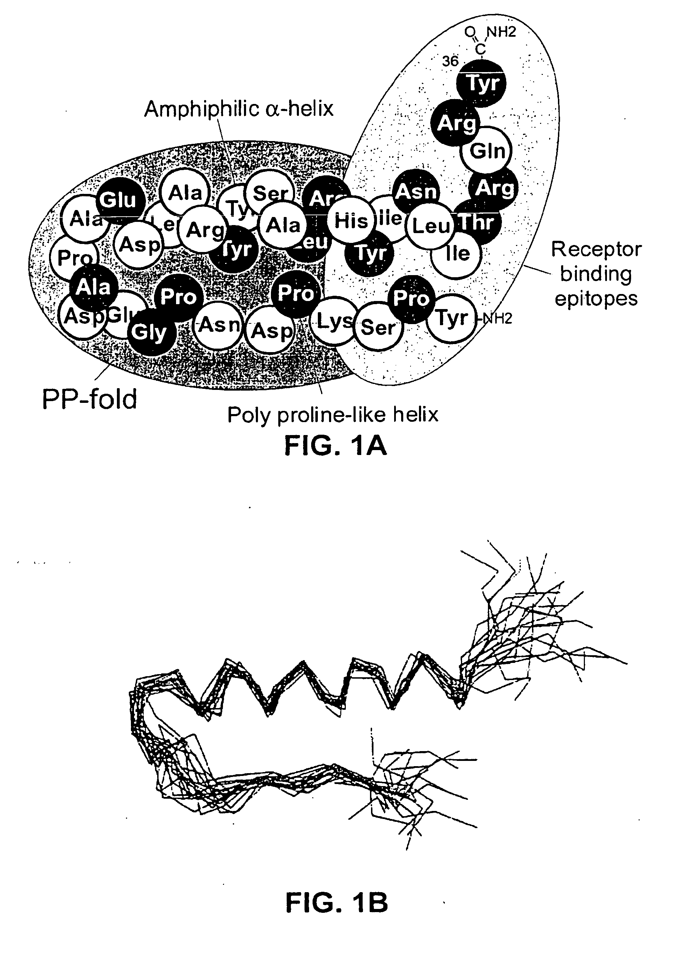

The PP-fold structure common to NPY, PYY and PP consists of 1) an N-terminal polyproline-like helix (corresponding to residues 1 through 8 with Pro2, Pro5, and Pro8) followed by 2) a type I beta-turn region (corresponding to residues 9 through 12) followed by 3) an amphiphilic alpha-helix (residues 13-30) which lies anti-parallel to the polyproline helix with an angle of about 152 degrees between the helical axes, and 4) a C-terminal hexapeptide (residues 31-36). The folded structure is stabilized through hydrophobic interactions between side chains of the amphiphilic alpha-helix which are closely interdigitating with the three hydrophobic proline residues (*Schwartz et al 1990*). Besides key residues in the receptor recognizing C-terminal hexapeptide it is the core hydrophobic residues, which stabilize the PP-fold structure, which are conserved across the family of PP-fold peptides. Fig. 1A depicts the NPY sequence, with residues which are conserved amongst NPY, PYY and PP shown as white text on dark background. Fig. 1A also illustrates the elements of the PP-fold structure described above. The C-terminal hexapeptide, which is important for receptor recognition is unstructured, but the PP fold provides a stable scaffold, which presents the C-terminal hexapeptide to the receptors (illustrated in Fig. 1 B), which to variable degree are dependent or independent also upon parts of the N-terminus of the peptides. NMR spectroscopic analysis has demonstrated that the far C- and the N-terminal parts of for example NPY are rather mobile, meaning that the PP-fold is constantly in danger of being "unzipped" from the free terminal end.

**NPY** is a very wide-spread neuropeptide with multiple actions in various parts of both the central and peripheral nervous system acting through a number of different receptor subtypes in man: Y1, Y2, Y4 and Y5. The main NPY receptors are the Y1 receptor, which generally is the post-synaptic receptor conveying the "action" of the NPY neurones and the Y2 receptor which generally is a pre-synaptic, inhibitory receptor. This is also the case in the hypothalamus, where NPY neurones - which also express the melanocortin receptor antagonist / inverse agonist AgRP (agouti related peptide) - act as the primary "sensory" neurones in the stimulatory branch of the arcuate nucleus. Thus, in this the "sensor nucleus" for the control of appetite and energy expenditure, the NPY/AgRP neurones together with the inhibitory POMC/CART neurones monitor the hormonal and nutritional status of the body as these neurones are the target for both the long-term regulators such as leptin and insulin and short term regulators such as ghrelin and PYY (see below). The stimulatory NPY/AgRP neurones project for example to the paraventricular nucleus - also of the hypothalamus - where its postsynaptic target receptors are believed to be Y1 and Y5 receptors. NPY is the most potent compound known in respect of increasing food intake, as rodents upon intracerebroventricular (ICV) injection of NPY will eat until they literally burst. AgRP from the NPY/AgRP neurones acts as an antagonist mainly on melanocortin receptors type 4 (MC-4) and block the action of POMC derived peptides - mainly aMSH - on this receptor. Since the MC4 receptor signal acts as an inhibitor of food intake, the action of AgRP is - just like the NPY action - a stimulatory signal for food intake (i.e. an inhibition of an inhibition). On the NPY/AGRP neurons are found inhibitory - pre-synaptic - Y2 receptors, which are the target both of locally released NPY as well as a target for the gut hormone PYY - another PP-fold peptide.

**PYY** is released during a meal - in proportion to the calorie content of the meal - from entero-endocrine cells in the distal small intestine and the colon, to act both in the periphery on GI-tract functions and centrally as a satiety signal. Peripherally, PYY is believed to function as an inhibitor - an "illeal break" - on for example upper GI-tract motility, gastric acid and exocrine pancreatic secretion. Centrally, PYY is believed to act mainly on the presynaptic, inhibitory Y2 receptors on the NPY/AgRP neurones in the arcuate nucleus, which it is believed get access to from the blood *(*Batterham et al. 2002 Nature 418: 650-4*).* The peptide is released as PYY1-36, but a fraction - approximately 50 % - circulates as PYY3-36 which is a product of degradation by dipeptidylpeptidase-IV an enzyme which removes a dipeptide from the N-terminus of a peptide provided that a Pro or Ala is found in position two as in all three PP-fold peptides - PP, PYY and NPY *(*Eberlein et al. 1989 Peptides 10: 797-803*).* Thus PYY in the circulation is a mixture of PYY1-36, which acts on both Y1 and Y2 receptors (as well as Y4 and Y5 with various affinities), and PYY3-36 - which has lower affinities for the Y1, Y4 and Y5 receptors than for the Y2 receptor.

**PP** is a hormone, which is released from endocrine cells in the pancreatic islets, almost exclusively governed by vagal cholinergic stimuli elicited by especially food intake (Schwartz 1983 Gastroenterology 85:1411-25). PP has various effects on the gastrointestinal tract, but none of these are observed in isolated cells and organs, and all appear to be dependent on an intact vagal nerve supply (Schwartz 1983 Gastroenterology 85:1411-25). In accordance with this, the PP receptors, which are called Y4 receptors, are located in the brain stem with a strong expression in vagal motor neurones - activation of which results in the peripheral effects of PP - and in the nucleus tractus solitarirus (NTS) - activation of which results in the effects of PP as a satiety hormone (Whitecomb et al. 1990 Am.J.Physiol. 259: G687-9 1*,* Larsen & Kristensen 1997 Brain Res.Mol.Brain Res 48: 1-6). It should be noted that PP from the blood has access to this area of the brain since the blood brain barrier is "leaky" in this area where various hormones from the periphery are sensed. Recently it has been argued that part of the effect of PP on food intake is mediated through an action on neurones - especially the POMC/CART neurones in the arcuate nucleus (Batterham et al. 2004 Abstract 3.3 International NPY Symposium in Coimbra, Portugal). PP acts through Y4 receptors for which it has a subnanomolar affinity as opposed to PYY and NPY which have nanomolar affinity for this receptor (Michel et al. 1998 Pharmacol. Rev. 50: 143-150). PP also has an appreciable affinity for the Y5 receptor, but it is not likely of physiological importance in relation to circulating PP due to both lack of access to the cells in the CNS where this receptor especially is expressed and due to the relatively low affinity for PP.

### PP-fold peptide receptors

There are four well established types of PP-fold peptide receptors in man: Y1, Y2, Y4, and Y5 which all recognize NPY1-36 and PYY1-36 with similar affinity. At one time a Y3 receptor type, which might prefer NPY over PYY, was suggested, but today this is not accepted as a real receptor subtype (Michel et al. 1998 Pharmacol. Rev. 50: 143-150). A Y6 receptor subtype has been cloned, which in man is expressed in a truncated form lacking TM-VII as well as the receptor tail and consequently at least on its own does not appear to form a functional receptor molecule.

**Y1 receptors** - affinity studies suggest Y1 binds NPY and PYY equally well and basically not PP. Affinity for Y1 is dependent on the identities of both end sequences of the PP-fold molecule (NPY/PYY) - for example residues Tyr1 and Pro2 are essential - and it is dependent on the peptide ends being presented in just the right way. In the C-terminal end, where the side-chains of several of the residues are essential, the Y1 receptor - like the Y5 and Y4 receptor but not the Y2 receptor - tolerates certain substitutions in position 34 (normally a Gln) - such as Pro (Fuhlendorff et al. 1990 J. Biol. Chem. 265: 11706-12*,* Schwartz et al. 1990 Annals NY Acad.Sci. 61: 35-47). Some structure-function studies concerning the requirements of the Y1 and Y2 receptors have been reported (Beck-Sickinger et al. 1994 Eur.J.Biochem. 225: 947-58*;* Beck-Sickinger and Jung 1995 Biopolymers 37: 123-42*;* S611 et al. 2001 Eur.J.Biochem. 268:2828-37).

**Y2 receptors** - affinity studies suggest Y2 binds NPY and PYY equally well and basically not PP. The receptor requires especially the C-terminal end of the PP-fold peptide (NPY/PYY). Thus, long C-terminal fragments - down to for example NPY13-36 (the whole alpha helix plus the C-terminal hexapeptide) - are recognized with relatively high affinity, i.e. to within ten-fold of the affinity of the full-length peptide (Sheikh et al. 1989 FEBS Lett. 245: 209-14*,* Sheikh et al. 1989 J. Biol. Chem. 264: 6648-54). Therefore various N-terminal deletions, which eliminate the binding to the Y1 receptor, still preserve some degree of binding to the Y2 receptor. However, the affinity of the C-terminal fragments is reduced approximately 10 fold as compared to NPY / PYY for even relatively long fragments. The Gln residue in position 34 of NPY and PYY is highly important for the ligand recognition of the Y2 receptor (Schwartz et al. 1990 Annals NYAcad.Sci. 611: 35-47).

**Y4 receptors** - affinity studies suggest that Y4 binds PP with subnanomolar affinity corresponding to the concentrations found in plasma whereas NPY and PYY are recognized with much lower affinity. Such studies suggest the Y4 receptor is highly dependent on the C-terminal end of the PP-fold peptides, and that relatively short N-terminal deletions impairs the affinities of the ligands. Some structure activity studies concerning the Y4 receptor have been reported *(*Gehlert et al. 1996 Mol.Pharmacol.50: 112-18*;* Walker et al. 1997 Peptides 18: 609-12*).*

**Y5 receptors** - affinity studies suggest that Y5 binds NPY and PYY equally well, and also binds PP with lower affinity, which however is below the normal circulating levels of this hormone. PYY3-36 is also recognized well by the Y5 receptor, however this receptor is to a large degree expressed in the CNS where such peptide cannot get access to the receptor readily when administered in the periphery.

PP-fold peptides and analogs of these have been suggested for use in the treatment of obesity and associated diseases, including for example Prader Willi's syndrome, based on the demonstrated effects of certain of the these peptides in animal models and in man and on the fact that obese people have low basal levels of PP and PYY as well as lower meal responses of these peptides (Holst JJ et al. 1983 Int.J.Obes. 7: 529-38*;* Batterham et al. 1990 Nature). It has been known since the mid seventies that PP could affect food intake in rodents. In 1993 it was reported that infusion of PP in morbidly obese patients with Prader Willi's syndrome decreased food intake (Berntson et al. 1993 Peptides 14: 497-503). Recently this effect of PP was confirmed by infusion of PP in normal human subjects where a long lasting suppression of appetite and reduced food intake over 24 hours was observed (Batterham et al 2003, Clin.Endocrinol.Metab. 88: 3989-92).

However, the native PP-fold peptides are not optimal for use as biopharmaceuticals, for example they are susceptible to degradation by various proteases such as DPP-IV. Thus, the natural peptides are not optimized for protein stability as they are made to normally act for a relatively short time as a neuropeptide or hormone.

For the treatment of conditions responsive to Y4 receptor modulation, such as obesity and intestinal hyper-secretion it would therefore be desirable to use PP-fold peptides or PP-fold peptide mimics, which acted as agonists and were specific for the Y4 receptor intended as target, and which stably preserve elements of the PP-fold structure important for receptor binding. In particular, it would be highly desirable to use such agents which are selective for the Y4 receptor over the Y1 and Y2 receptors. This is particularly important, since activation of the Y1 receptor is expected to potentially cause unwanted cardiovascular and renal side effects such as vasoconstriction and natriuresis Moreover, activation of the Y2 receptor may also cause side effects. Although it is still unclear what the really efficient angiogenic Y receptor profile is, Y2 agonists such as NPY3-36 apparently can induce revascularization in for example ischemic hind limb models, i.e. when administered in high doses with constant exposure as for example released from inoperated pellets *(*Zukowska Z et al. Trends Cardiovasc Med. 2003 ,13 :86-92*).* The angiogenic response to NPY is reduced in Y2 receptor knock out animals; however, the response to this broad-spectrum Y receptor agonist NPY is in fact not eliminated and both Y2 and Y5 receptors are up-regulated in ischemic vessels *(*Lee et al J.Clin.lnvest. 2003, 111: 1853-62*).* Nevertheless, a PP-fold peptide or PP-fold peptide mimic could through activation of the Y2 receptor cause side effects such as worsen the retinopathy for example in diabetic patients and could potentially aid in the neovascularization associated with the growth of certain cancers. Thus use of efficacious and selective Y4 receptor over Y1 and Y2 receptor agonists would be particularly useful in diseases and conditions susceptible to Y4 receptor activation.

### Some Common Terms Used In this Specification

**Affinity:** The affinity of a peptide to a specific receptor is given for example as an IC₅₀ value or a Kᵢ or K_{d} value, which in a specific, non-limiting example is determined in an assay, such as a competition binding assay. The IC50 value corresponds to the concentration of the peptide which displaces a - for the given receptor relevant - radioactive ligand used in an amount far less than the Kd for that radioactive ligand to 50%.

**Appetite:** A natural desire, or longing for food. Increased appetite generally leads to increased feeding behavior.

**Appetite Suppressants:** Compounds that decrease the desire for food

**Binding:** A specific interaction between two molecules, such that the two molecules interact. Binding to a receptor can be specific and selective, so that one molecule is bound preferentially when compared to another molecule. Specific binding may be identified by a disassociation constant (K_{d}). This value is dependent on the selectivity of the compound tested. For example, a compound with a K_{d} that is less than 10 nM is generally considered an excellent drug candidate. However, a compound that has a lower affinity, but is selective for the particular receptor, can also be a good drug candidate.

**Body Mass Index (BMI):** A mathematical formula for measuring body mass, also sometimes called Quetelet's Index. BMI is calculated by dividing weight (in kg) by height² (in meters). The current standards for both men and women accepted as "normal" are a BMI of about 20 kg/m². In one embodiment, a BMI of greater than 25 kg/m² can be used to identify an obese subject. Grade I obesity corresponds to a BMI of 25 kg/m². Grade II obesity corresponds to a BMI of 30-40 kg/m²; and Grade III obesity corresponds to a BMI greater than 40 kg/m² *(*Jequier 1987 Ain. J Clin. Nutr. 45:1035-47*).* Ideal body weight will vary among species and individuals based on height, body build, bone structure, and sex.

**Caloric intake or calorie intake:** The number of calories (energy) consumed by an individual. In the present context this term is identical to the term "energy intake".

**Cosmetic treatment:** The term is intended to denote a treatment that is not for medical purposes, but for improving the well-being of a subject e.g. with respect to the appearance of a subject. Included in the term is treatment of a subject who desires to decrease his weight without necessarily being overweight or obese.

**Food intake:** The amount of food consumed by an individual. Food intake can be measured by volume or by weight. Included within its meaning is i) food intake as being the total amount of food consumed by an individual, and ii) food intake is the amount of proteins, fat, carbohydrates, cholesterol, vitamins, minerals, or any other food component, of the individual. Accordingly, the term food intake as used in the present context is similar to the term "energy intake".

**Normal Daily Diet:** The average food intake for an individual of a given species. A normal daily diet can be expressed in terms of caloric intake, protein intake, carbohydrate intake, and/or fat intake. A normal daily diet in humans generally comprises the following: about 2,000, about 2,400, or about 2,800 to significantly more calories. In addition, a normal daily diet in humans generally includes about 12 g to about 45 g of protein, about 120 g to about 610 g of carbohydrate, and about 11 g to about 90 g of fat. A low calorie diet would be no more than about 85%, and preferably no more than about 70%, of the normal caloric intake of a human individual. In animals, the caloric and nutrient requirements vary depending on the species and size of the animal. For example, in cats, the total caloric intake per kg, as well as the percent distribution of protein, carbohydrate and fat varies with the age of the cat and the reproductive state.

**Obesity:** A condition in which excess body fat may put a person at health risk (see Barlow and Dietz, Pediatrics 102:E29, 1998*;* National Institutes of Health, National Heart, Lung, and Blood Institute (NHLBI), Obes. Res. 6 (suppl. 2):51 S209S, 1998*).* Excess body fat is a result of an imbalance of energy intake and energy expenditure. In one embodiment, the Body Mass Index (BMI) is used to assess obesity. In one embodiment, a BMI of from about 22 kg/m² (i.e. about 10% above the normal value) and up to about 30 kg/m² is regarded as overweight, especially from about 25.0 kg/m² and up to 30 kg/m², while a BMI of 30 kg/m² or more is obese.

**Overweight:** An individual who weighs more than their ideal body weight. An overweight individual can be obese, but is not necessarily obese. In one embodiment, an overweight individual is any individual who desires to decrease their weight. In another embodiment, overweight individual is an individual with a BMI of a BMI of from about 22 kg/m² (i.e. about 10% above the normal value) and up to about 30 kg/m² is regarded as overweight, especially from about 25.0 kg/m² and up to 30 kg/m². It should be noted that subjects having a BMI that is only slightly higher than that of the normal value (e.g. from about 22 to about 25 kg/m²) very often have a desire to loose weight although this may only be for cosmetic reasons.

**Potency:** In vitro potency of a compound is defined in terms of EC₅₀ values, i.e. the concentration that leads to 50% of the maximally achievable effect as determined in a for the given receptor relevant signaling assay.

**Subject:** The subject can be any subject, including both human and veterinary mammalian subjects. Thus, the subject can be a human, or can be a non-human primate, a farm animal such as swine, cattle, sheep and poultry, a sport animal or pet such as dogs, cats, horses, hamsters, and rodents.

**Therapeutically effective amount:** A dose sufficient to prevent, treat or ameliorate a specific condition or disease and/or to alleviate specific signs or symptoms of a specific condition or disease. The term includes a dose that is sufficient or prevent advancement, or to cause regression of a disorder, or which is capable of relieving a sign or symptom of a disorder, or which is capable of achieving a desired result. In embodiments relating to cosmetic treatment or to treatment of overweight or obesity, a therapeutically effect of a receptor agonist is an amount sufficient to inhibit or halt weight gain, or an amount sufficient to decrease appetite, or an amount sufficient to reduce energy or food intake or increase energy expenditure. The term "cosmetically effective amount" is intended to denote a dose that is sufficient for the subject being treated to achieve a desired effect.

### DETAILED DESCRIPTION OF THE INVENTION

In its broadest aspect, the present invention provides the use of a Y receptor agonist which is selective for the Y4 receptor over the Y1 and Y2 receptors, said Y receptor agonist being pancreatic peptide PP2-36 with SEQ ID No:10, in the preparation of a composition for (i) reducing food intake, (ii) treatment of obesity or overweight, (iii) treatment of diarrhoea or hypersecretion associated with small intestinal ileo-stomia or (iv) treatment of a condition in which obesity or overweight is considered a contributory factor, selected from bulimia, bulimia nervosa, Syndrome X (metabolic syndrome), diabetes, type 2 diabetes mellitus or Non Insulin Dependent Diabetes Mellitus (NIDDM), hyperglycemia, insulin resistance, impaired glucose tolerance, cardiovascular disease, hypertension, atherosclerosis, coronary artery disease, myocardial infarction, peripheral vascular disease, stroke, thromboembolic disease, hypercholesterolemia, hyperlipidemia, gallbladder disease, osteoarthritis, sleep apnea, polycystic ovarian syndrome, and cancer of the breast, prostate, or colon.. PP2-36 (SEQ ID No: 10), is referred to in Bard et. al, J. Biol. Chem. (1995) 270: 26762-26765 and Walker et. al. Peptides vol 18, no. 4, 609-612 (1997). However, neither reference discloses the use of the peptide for the purposes of this invention.

Gehlert et. al. Molecular Pharmacology 199607 US, Vol. 50, no. 1, July 1996 (1996-07), pp 112-118, includes receptor binding data concerning N-terminal truncations of bovine PP.

PP2-36 is **selective for the Y4 receptor over the Y1 and Y2 receptors.** In the present context, this condition is fulfilled if the agonist has an IC50 value that is at least 10-fold lower for the Y4 than for the Y1 and Y2 receptors when measured in the affinity assay described herein. With respect to potency, PP2-36 has an EC50 value at least 10-fold lower for the Y4 than for the Y1 and Y2 receptors when measured in the potency assay described herein.

PP2-36 is a highly selective Y4 receptor agonist with improved stability as compared to PP1-36. PP is normally a substrate for DPP-IV degradation as it has a Pro in position 2. Thus, by removing the first residue from PP a peptide is generated where the PP fold structure is not jeopardized because residue 1 (Ala1) does not make any interactions with the anti-parallel helix (this is taken care of by Pro2) and because PP2-36 no longer like the wild-type peptide is a substrate for DPP-IV. Importantly, the Y4 receptor is not dependent on the presence of a residue in position 1, in contrast the Y1 receptor is dependent on the presence of a residue in position 1. Thus, PP2-36 is both a more stable peptide and it is a selective Y4 receptor agonist which has an improved selectivity window towards the Y1 receptor.

*Receptor recognition profile-* PP2-36 binds with an affinity to the Y4 receptor, IC50 = 0.64 nM, which is similar to the affinity of PP, IC50 = 0.41. The affinity of PP2-36 on the Y1 and Y2 receptors is > 1000 nM (Table 1). PP2-36 acts as an agonist on the Y4 receptor with a potency of 0.64 nM (EC50) which also is very similar to the potency of PP (EC50 = 0.85 nM) as measure in the inositol phosphate turnover assay performed in transfected COS-7 cells as described in the experimental section (Table 2). Both PP2-36 and PP are low potency ligands for the Y2 receptor, i.e. with EC50 values > 1000 nM. However, on the Y1 receptor PP2-36 has a potency which is 3-4 fold lower than that of PP, EC50 values being 297 versus 83 nM (Table 2). Thus PP2-36 is a highly selective Y4 agonist with > 300 fold window to both the Y1 and the Y2 receptor as determined in a functional in vitro assay.

*Protein stability-* PP2-36 has the advantage as compared to PP1-36 that it is more stable and therefore a better biopharmaceutical agent as it is not a substrate for DPP-IV - as PP is due to the presence of a Pro residue in position 2 - and since the novel N-terminal Pro residue in PP2-36 prevents against degradation by most other amino peptidases. These stability properties are particularly useful in the context where a prolonged presence of the peptide in body fluids is obtained through various means such as motif modifications of the peptide as such or through various forms of pharmaceutical methods such as sustained release formulations.

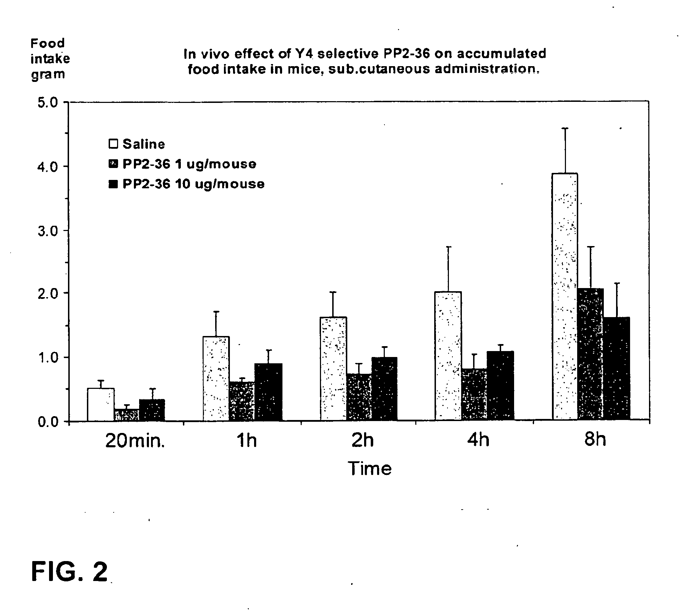

*In vivo effect on acute food intake -* Either saline or PP2-36 was administered by single subcutaneous injections to groups of 8 mice in doses of 1 and 10 µg per animal after 16 hours of fasting. In Fig. 2 is shown the accumulated food intake of the mice during 8 hours. The selective Y4 receptor agonist, PP2-36 inhibited food intake during the first 20 minutes after initiation of the meal to approx. 50 % of that ingested by the group of animals receiving saline. The 50 % suppressive effect on food intake was observed also when this was measured during the first full hour. The effect of 1 ug and 10 ug of PP2-36 was rather similar. The suppressive effect of PP2-36 was sustained for the full period of 8 hours after the single sub cutaneous injection (Fig 2). Thus PP2-36, which is highly selective for the Y4 over the Y2 and Y1 receptors is an efficient suppressor of food intake in mice as previously demonstrated for PP (Asakawa et al. 1999 Peptides 20: 1445-48, Asakawa 2003 Gastroenterology 124: 1325-36). It has previously been demonstrated that this effect of PP is also observed in normal human subjects (Batterham et al. 2003 Clin.Endocrinol.Metab. 88: 3989-92) as well as in mobidly obese patients (Berntson et al. 1993 Peptides 14: 497-503

### Clinical Indications

PP2-36 is of value in the treatment of conditions responsive to activation of Y4 receptors. Such conditions include those for which regulation of energy intake or energy metabolism is indicated.

Diseases or conditions in which regulation of energy intake or energy metabolism is indicated include obesity and overweight, and conditions in which obesity and overweight are considered contributory factors, such as bulimia, bulimia nervosa, Syndrome X (metabolic syndrome), diabetes, type 2 diabetes mellitus or Non Insulin Dependent Diabetes Mellitus (NIDDM), hyperglycemia, insulin resistance, impaired glucose tolerance, cardiovascular disease, hypertension, atherosclerosis, coronary artery disease, myocardial infarction, peripheral vascular disease. stroke, thromboembolic diseases, hypercholesterolemia, hyperlipidemia, gallbladder disease, osteoarthritis, sleep apnea, reproductive disorders such as polycystic ovarian syndrome, or cancer of the breast, prostate, or colon.

PP2-36 is also of value in the treatment of diarrhoea or hyper-secretion from intestinal stomia.

### 1. Obesity and Overweight

It was suggested already in the seventies that PP might be involved in the control of food intake. Recently, much evidence from rodent studies has accumulated showing very clearly that PP is in fact a powerful and efficient anorexigenic peptide when administered peripherally (Asakawa et al. Peptides 1999, 20; 1445-8; Katsuura et al. Peptides 2002, 23: 323-9; Asakawa et al. Gastroenterology 2003, 124: 1325-36). Since PP has no effect on appetite, food intake etc. in Y4 knock out animals it is very likely that PP acts through the Y4 receptor to reduce appetite and food intake (Batterham et al. 2004 abstract S3.3 from International NPY symposium in Coimbra Portugal). PP also has effect on food intake in diet induced obese animals. PP receptors have been found especially in the brain stem in area postreama and on vagal motor neurones where the blood brain barrier is not efficient and where circulating hormones such as PP can get access to the neurones. Thus it is very likely that the Y4 receptors in the NTS in the brain stem are a major target through which PP acts to suppress appetite and food intake. However, recent evidence also points to the possibility that PP may also act through Y receptors in the arcuate nucleus conceivably on the POMC and perhaps also the NPY/ AgRP neurones (Batterham et al. Coimbra NPY meeting abstract S3.3). Low levels of PP are found in obese subjects especially Prader-Willi syndrome (Zipf et al. J.C.E.M.1981, 52: 1264-6, Holst et al 1983, Int.J.Obes. 7: 529-38, Glaser et al Horm.Metab. 1988, 20: 288-92) and high PP levels are found in patients with anorexia nervosa. Importantly, infusion of PP in man decreases appetite and food intake for up to 24 hours (Batterham et al. JCEM 2003, 88: 3989-92). Thus, the effect of PP on food intake was observed after the PP levels in the circulation had returned to normal levels. Such long lasting effects on appetite etc, is well know from other compounds for example also from ICV injection of AgRP. Importantly infusion of PP has been shown to decrease food intake in mobidly obese patients with Prader Willi syndrome (Berntson et al 1993 Peptides 14: 497-503).

Hence, PP2-36 is suitable for use in a subject, such as a mammal including a human, in order to regulate the energy intake. Accordingly, PP2-36 may be used for altering energy intake, food intake, appetite, and energy expenditure.
Administration of PP2-36 results in a decrease in the amount, either the total weight or the total volume or calorie content of the food and/or a decrease of the intake of a food component, such as a decrease in the ingestion of lipids, carbohydrates, cholesterol, or proteins. Appetite may be reduced by administering a therapeutically effective amount of PP2-36. Appetite can be measured by any means known to one of skill in the art.

For example, decreased appetite can be assessed by a psychological assessment. In such an embodiment, administration of the receptor agonist results in a change in perceived hunger, satiety, and/or fullness. Hunger can be assessed by any means known to one of skill in the art. In one embodiment, hunger is assessed using psychological assays, such as by an assessment of hunger feelings and sensory perception using e.g. a questionnaire.

PP2-36 may be used for decreasing the motility of the upper GI tract as for example decreasing gastric emptying. PP, the prototype Y4 agonist is known to decrease gastric emptying. A therapeutically effective amount of PP2-36 may be administered to the subject, thereby decreasing GI-tract motility. It is well known that compounds which decrease gastric emptying will have a beneficial effect in also decreasing food intake as the subject is feeling more full or satiated.

PP2-36 may be used for altering energy metabolism in a subject. Energy is burned in all physiological processes. The body can alter the rate of energy expenditure directly, by modulating the efficiency of those processes, or changing the number and nature of processes that are occurring. For example, during digestion the body expends energy moving food through the bowel, and digesting food, and within cells, the efficiency of cellular metabolism can be altered to produce more or less heat. In a further embodiment a method is disclosed herein for any and all manipulations of the arcuate circuitry described in this application, which alter food intake coordinately and reciprocally alter energy expenditure. Energy expenditure is a result of cellular metabolism, protein synthesis, metabolic rate, and calorie utilization. Thus, peripheral administration of PP2-36 results in increased energy expenditure, and decreased efficiency of calorie utilization.

PP2-36 can be used for weight control and treatment, reduction or prevention of obesity, in particular any one or more of the following: preventing and reducing weight gain; inducing and promoting weight loss; and reducing obesity as measured by the Body Mass Index. As mentioned above, the invention also relates to the use of the PP2-36 for controlling any one or more of appetite, satiety and hunger, in particular any one or more of the following: reducing, suppressing and inhibiting appetite; inducing, increasing, enhancing and promoting satiety and sensations of satiety; and reducing, inhibiting and suppressing hunger and sensations of hunger. The disclosure further relates to the use of PP2-36 in maintaining any one or more of a desired body weight, a desired Body Mass Index, a desired appearance and good health.

PP2-36 may be used for the treatment and/or prevention of feeding disorders, appetite disorders, overweight, obesity, bulimia, bulimia nervosa, Syndrome X (metabolic syndrome), or complications or risks associated thereto including diabetes, type 2 diabetes mellitus or Non Insulin Dependent Diabetes Mellitus (NIDDM), hyperglycemia, insulin resistance, impaired glucose tolerance, cardiovascular disease, hypertension, atherosclerosis, congestive heart failure, stroke, myocardial infarct, thromboembolic diseases, hypercholesterolemia, hyperlipidemia, gallbladder disease, osteoarthritis, sleep apnea, reproductive disorders such as polycystic ovarian syndrome, cancers of the breast, prostate, and colon, the method comprising administering to a subject such as a mammal including a human, an effective dose of PP2-36 as described herein.

### 2. Intestinal hypersecretion

PP is know to have a strong anti-secretory effect on both the small and large intestine and this appears to be mediated through Y4 receptors located on the epithelial cells (Cox & Tough 2001 Br.J.Pharmacol. 135: 1505-12). It has been shown in vivo that peripheral administration of PYY - another PP-fold peptide activating Y1 and Y2 receptors - can cause a long-lasting reduction in intestinal secretion induced by vasoactive intestinal polypeptide in human subjects with ileostomies (Playford et al 1990 Lancet 335: 1555-57). It was concluded that PYY could be a therapeutic agent against diarrhoea. However, for example the natriuretic and hypertensive effects of the combined Y1 and Y2 agonists, NPY and PYY peptides have prevented this. Such side effects will not be relevant for PP2-36. Thus PP2-36 is particularly useful for the treatment or protection against hyper-secretion of the GI-tract including various forms of diarrhoea whether or not they directly are caused by hyper-secretion as a suppression of the intestinal secretion will either eliminate the cause of the diarrhoea or eliminate the symptoms. One particularly interesting indication is the hyper-secretion observed in patients with ileostomia, who often are losing large amounts of fluid. PP2-36 is particularly useful for the treatment or protection against hypersecretion associated with small intestinal ileo-stomia.

### Additional comments concerning administration of PP2-36 for the treatment or prevention of obesity and related diseases

During a meal a large repertoire of gastrointestinal hormones and neurotransmitter systems are activated in a carefully concerted, sequential and overlapping manner. Moreover, food components influence not only the secretion of GI hormones and the activity of various afferent neuronal pathways but these food components also influence various hormones and centers in the CNS directly after they are absorbed. Thus the regulation of food intake and energy expenditure is a highly complex and multifaceted process. In view of this it is surprising that certain hormones such as PP in fact can substantially affect the system when administered in a way which results in, for example only 3-4 times the plasma levels which are achieved during a meal.

Administration of the PP2-36 agonist apparently mainly has the intended effect if the compound is given in the fasting state in an effective dose as described. If the agonist is given in a situation where the various hormonal and neuronal systems are active due to the presence of food components in the GI tract or the expectation of a meal, the effect is not seen or a smaller effect is observed. Thus, the selective Y4 agonist is preferably administered in the fasting state in an effective dose either subcutaneously, nasally or through other means as described elsewhere herein. In the present context, the term "fasted state" means that the subject has not eaten any food or drink within at least the last 2 hours before administration of the Y2 receptor agonist such as, e.g., within at least the last 3 hours, within at least the last 4 hours, within at least the last 5 hours, within at least the last 6 hours, within at least the last 7 hours, within at least the last 8 hours, within at least the last 9 hours, within at least the last 10 hours, within at least the last 11 hours or within at least the last 12 hours before dosing.

In a subgroup of the population, PP2-36 may not have the intended action due to genetic variations such as polymorphisms in the Y4 receptor gene. Loss of function mutations in these receptors are likely to be associated with obesity. Thus, in a preferred embodiment of the invention an analysis of the Y4 gene of the subject to be treated is performed in order to probe for polymorphisms / mutations in these genes and identification of such polymorphisms. Based on such an analysis an optimal treatment of the subjects can be made. For example, only subjects with normal genotype or with polymorphisms, which do not affect the function of the Y4 agonist, should be treated with PP2-36 . Another possibility is to increase the dose of PP2-36 in subjects who express an impaired receptor in order to ensure an optimal effect of the drug. In the case where the obesity of a subject is caused by an impairment in the function of the Y4 receptor it could be argued that treatment with a - for example large doses - of PP2-36 is a form of replacement therapy - provided that at least some of the relevant receptor function is still left - for example in heterozygote patients.

An acute test may be performed where PP2-36 is administered to ensure that it has the intended effect in the subject to be treated before a chronic treatment is started. Through these means it is ensured that only subjects who are susceptible to treatment with PP2-36 are treated with the compound.

PP2-36 may be combined in the treatment of obesity, diabetes and related diseases with the use of various other drugs targeting appetite and energy expenditure. This includes but is not limited to drugs such as GI-tract lipase inhibitors, neurotransmitter reuptake inhibitors, cannabinoid receptors antagonists and inverse agonists, as well as other types of neurotransmitter - including but not limited to 5HT receptors - and/or hormone - including but not limited to GLP-1, MC4, MC3 - receptor agonist or antagonists. Due to the fact that the Y4 selective agonist is targeting a homeostatic regulatory mechanism in the communication between the GI-tract and the CNS - i.e. the Y4 receptors normally targeted by the satiety mediating hormone PP from the pancreas - it is particularly beneficial to combine the treatment with PP2-36 with the treatment with a drug targeting a central, hedonic mechanism in the regulation of appetite and energy expenditure, such as the CB1 receptors, for example being part of the reward system.

### Dosages

The therapeutically effective amount of PP2-36 will be dependent on the age, weight and condition of subject being treated, the severity and type of the condition or disease being treated, the manner of administration and the strength of the composition applied.

For example, a therapeutically effective amount of PP2-36 can vary from about 0.01 µg per kilogram (kg) body weight to about 1 g per kg body weight, such as about 1 µg to about 5 mg per kg body weight, or about 5 µg to about 1 mg per kg body weight. In another embodiment, PP2-36 is administered to a subject at 0.5 to 135 picomole (pmol) per kg body weight, or about 72 pmol per kg body weight.

In one specific, non-limiting example from about 5 to about 50 nmol is administered as a subcutaneous injection, such as from about 2 to about 20 nmol, or about 1.0 nmol is administered as a subcutaneous injection. The exact dose is readily determined by one skilled in the art based on the potency of the specific compound (such as the receptor agonist) utilized, the age, weight, sex and physiological condition of the subject. The dose of an agonist can be a molar equivalent of the therapeutically effective dose of PYY3-36.

The amounts can be divided into one or several doses for administration daily, every second day, weekly, every two weeks, monthly or with any other suitable frequency. Normally, the administration is once or twice daily.

### Methods of administration

PP2-36 as well as pharmaceutical compositions according to the invention can be administered by any route, including the enteral (e.g. oral administration) or parenteral route. In a specific embodiment, the parenteral route is preferred and includes intravenous, intraarticular, intraperitoneal, subcutaneous, intramuscular, intrasternal injection and infusion as well as administration by the sublingual, transdermal, topical, transmucosal including nasal route, or by inhalation such as, e.g., pulmonary inhalation. In specific embodiments, the subcutaneous and/or the nasal administration route is preferred.

When administered centrally, the natural Y4 selective peptide PP may, as do NPY and PYY when administered ICV, induce eating (probably due to activation of central receptors which normally are not reached by the circulating hormones or peripherally administered peptide compounds). Thus in cases where increased eating is to be avoided, it is preferred that PP2-36 is administered peripherally.

PP2-36 can be administered as such dispersed in a suitable vehicle or they can be administered in the form of a suitable pharmaceutical composition. Such compositions are also within the scope of the invention. In the following are described suitable pharmaceutical compositions.

### Pharmaceutical compositions

The PP2-36 for use in medicine is normally presented in the form of a pharmaceutical composition comprising PP2-36 together with one or more physiologically or pharmaceutically acceptable excipients.

PP2-36 may be administered to an animal including a mammal such as, e.g., a human by any convenient administration route such as, e.g., the oral, buccal, nasal, ocular, pulmonary, topical, transdermal, vaginal, rectal, ocular, parenteral (including inter alia subcutaneous, intramuscular, and intravenous cf. above), route in a dose that is effective for the individual purposes. A person skilled in the art will know how to chose a suitable administration route. As mentioned above, the parenteral administration route is preferred. In a specific embodiment, the receptor agonist is administered subcutaneously and/or nasally. It is well known in the art that subcutaneous injections can be easily self-administered.

A composition suitable for a specific administration route is easily determined by a medical practitioner for each patient individually. Various pharmaceutically acceptable carriers and their formulation are described in standard formulation treatises, e.g., Remington's Pharmaceutical Sciences by E. W. Martin.

The pharmaceutical composition comprising PP2-36 may be in the form of a solid, semi-solid or fluid composition. For parenteral use, the composition is normally in the form of a fluid composition or in the form of a semi-solid or solid form for implantation.

Fluid compositions, which are sterile solutions or dispersions can utilized by for example intravenous, intramuscular, intrathecal, epidural, intraperitoneal or subcutaneous injection of infusion. PP2-36 may also be prepared as a sterile solid composition, which may be dissolved or dispersed before or at the time of administration using e.g. sterile water, saline or other appropriate sterile injectable medium.

The fluid form of the composition may be a solution, an emulsion including nano-emulsions, a suspension, a dispersion, a liposomal composition, a mixture, a spray, or a aerosol (the two latter types are especially relevant for nasal administration).

Suitable mediums for solutions or dispersions are normally based on water or pharmaceutically acceptable solvents e.g. like an oil (e.g. sesame or peanut oil) or an organic solvent like e.g. propanol or isopropanol. A composition according to the invention may comprise further pharmaceutically acceptable excipients such as, e.g., pH adjusting agents, osmotically active agents e.g. in order to adjust the isotonicity of the composition to physiologically acceptable levels, viscosity adjusting agents, suspending agents, emulsifiers, stabilizers, preservatives, antioxidants etc. A preferred medium is water.

Compositions for nasal administration may also contain suitable non-irritating vehicles such as, e.g., polyethylene glycols, glycofurol, etc. as well as absorption enhancers well known by a person skilled in the art (e.g. with reference to Remington's Pharmaceutical Science)

For parenteral administration, in one embodiment PP2-36 can be formulated generally by mixing it at the desired degree of purity, in a unit dosage injectable form (solution, suspension, or emulsion), with a pharmaceutically acceptable excipient or carrier, i.e., one that is non-toxic to recipients at the dosages and concentrations employed and is compatible with other ingredients of the composition.

Generally, the formulations are prepared by contacting PP2-36 uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes. Due to the amphiphatic nature of the peptides described herein suitable forms also include micellar formulations, liposomes and other types of formulations comprising one or more suitable lipids such as, e.g., phospholipids and the like.

Preferably, they are suspended in an aqueous carrier, for example, in an isotonic buffer solution at a pH of about 3.0 to about 8.0, preferably at a pH of about 3.5 to about 7.4, 3.5 to 6.0, or 3.5 to about 5. Useful buffer substances include acetate, citrate, phosphate, borate, carbonate such as, e.g., sodium citrate-citric acid and sodium phosphate-phosphoric acid, and sodium acetate/acetic acid buffers.

The compositions may also be designed to controlled or prolonged delivery of the receptor agonist after administration in order to obtain a less frequent administration regimen. Normally a dosage regimen including 1-2 daily administrations is considered suitable, but within the scope of the present invention is also included other administration regimens such as, e.g., more frequent and less frequent. In order to achieve a prolonged delivery of PP2-36, a suitable vehicle including e.g. lipids or oils may be employed in order to form a depot at the administration site from which PP2-36 is slowly released into the circulatory system, or an implant may be used. Suitable compositions in this respect include liposomes and biodegradable particles into which PP2-36 has been incorporated.

In those situations where solid compositions are required, the solid composition may be in the form of tablets such as, e.g. conventional tablets, effervescent tablets, coated tablets, melt tablets or sublingual tablets, pellets, powders, granules, granulates, particulate material, solid dispersions or solid solutions.

A semi-solid form of the composition may be a chewing gum, an ointment, a cream, a liniment, a paste, a gel or a hydrogel.

Other suitable dosages forms of the pharmaceutical compositions according to the invention may be vagitories, suppositories, plasters, patches, tablets, capsules, sachets, troches, devices etc.

The dosage form may be designed to release PP2-36 freely or in a controlled manner e.g. with respect to tablets by suitable coatings.

The pharmaceutical composition may comprise a therapeutically effective amount of PP2-36.

The content of PP2-36 in a pharmaceutical composition of the invention is e.g. from about 0.1 to about 100% w/w of the pharmaceutical composition.

The pharmaceutical compositions may be prepared by any of the method well known to a person skilled in pharmaceutical formulation.

In pharmaceutical compositions, PP2-36 is normally combined with a pharmaceutical excipient, i.e. a therapeutically inert substance or carrier.

The carrier may take a wide variety of forms depending on the desired dosage form and administration route.

The pharmaceutically acceptable excipients may be e.g. fillers, binders, disintegrants, diluents, glidants, solvents, emulsifying agents, suspending agents, stabilizers, enhancers, flavours, colors, pH adjusting agents, retarding agents, wetting agents, surface active agents, preservatives, antioxidants etc. Details can be found in pharmaceutical handbooks such as, e.g., Remington's Pharmaceutical Science or Pharmaceutical Excipient Handbook.

### Syntheses

PP2-36 may be synthesized by solid phase peptide synthesis, using either an automated peptide synthesizer, or traditional bench synthesis. The solid support can be, for example, chlorotrityl (CI) or Wang (OH) resin, both of which are readily available commercially. The active groups of those resins react readily with the carboxyl group of an N-Fmoc amino acid, thereby covalently binding it to the polymer. The resin-bound amine may be deprotected by exposure to piperidine. A second N-protected amino acid may then be coupled to the resin-amino acid. These steps are repeated until the desired sequence is obtained. At the end of the synthesis, the resin-bound protected peptide may be deprotected and cleaved from the resin with trifluoroacetic acid (TFA). Examples of reagents facilitating the coupling new amino acids to the resin-bound amino acid chain are: tetra-methyluronium hexafluorophosphate (HATU), O-(1H-benzotriazole-1-yl)-N,N,N',N'-tetramethyluronium hexafluorophosphate (HBTU), O-(1H-benzotriazole-1-yl)-N,N,N',N'-tetramethyluronium tetrafluoroborate (TBTU), 1H-hydroxybenzotriazole (HOBt).

Peptide synthesis by solution chemistry rather than solid phase chemistry is also feasible.

PP2-36 was made by solid phase synthesis, on PAL Peg-PS resin, (amide resin (amide resin Applied Bioscience, Warrington, UK GEN913401), using Fmoc chemistry with a 5x reagent excess. The coupling was performed by HCTU throughout, solvent DMF. Fmoc removal was performed with 20% piperidine in DMF, 10-15 minutes. However, the peptide could just as well have been synthesised by various other standard peptide synthesis methods such as tBOC chemistry and solution chemistry instead of solid state etc. The synthesis is illustrated by the following description, but PP2-36 was made by similar methods:

### Illustrative Synthesis of [Cys2, D-Cys27]PP (SEQ IS No: 4)

In general side group protection were standard Fmoc except for:

Arg = Fmoc Arg(Pbf) -OH

Asn, Gln = Fmoc Asn(Trt)-OH

Thr, Ser, Asp, Glu, Tyr = tButyl

Ala-Ser 22-23 = Fmoc AlaSer pseudoproline

In the case of [Cys2, D-Cys27]PP the following special protection groups were used in order to obtain selective deprotection:

Cys 27 = Fmoc DLys(Trt) -OH

The peptide was synthesized by solid phase synthesis, on PAL Peg-PS resin (a resin which will generate the biologically important carboxyamide group upon cleavage), using Fmoc chemistry with a 5 fold molar reagent excess. The coupling was performed by HCTU throughout using DMF as solvent. Fmoc removal after each coupling step was performed with 20% piperidine in DMF for 10-15 minutes. The coupling was checked after each step by quantitative ninhydrin assay. In certain cases double couplings could be performed.

The resin was subsequently divided into different batches to generate the peptide.

| ***Structure*** | **Molecular formula** | **Mw** | **Theoretical m/z** | **Measured Mass m/z** | **Rt min** | **Purity %** | **Analytical method** |
|---|---|---|---|---|---|---|---|
| PP2-36 | C182H282N5 205352 | 4110.7 | | 4109.2 | 18.1 | 96.2 | C |
| [Cys2,D-Cys27]-PP | C177H279N5 305354 | 4125.8 | | 4124.4 | 17.9 | 98.7 | C |

### Analvtical HPLC method C

| | |
|---|---|
| Column | = Vydac C18 218TP54, 250 x 4.6 mm |
| Buffer A | = 20 mL of MeCN and 2 mL of TFA in Water (total volume 2000 mL) |
| Buffer B | = 2 mL of TFA in Water (total volume 2000 mL) |
| Gradient | = 25% B to 75% B over 27 min |
| Flow rate | = 1.00 mL/min |
| Wavelength | = 215 nm |
| | |
| Injection volume | = 10 µL |

### Biological Assays and Results

### I. IN VITRO ASSAYS TO DETERMINE PEPTIDE AFFINITY AND POTENCY

### Human Y2 receptor Affinity Assay

Affinity of test compounds for the human Y2 receptor is determined in a competition binding assay using 1251-PYY binding in COS-7 cells transiently transfected with the human Y2 receptor using a standard calcium phosphate transfection method.

Transfected COS-7 cells are transferred to culture plates one day after transfection at a density of 40 x 103 cells per well aiming at 5 - 8 % binding of the radioactive ligand. Two days after transfection, competition binding experiments are performed for 3 hours at 4 C° using 25 pM of 125I-PYY (Amersham, Little Chalfont, UK). Binding assays are performed in 0.5 ml of a 50 mM Hepes buffer, pH 7.4, supplemented with 1 mM CaCl2, 5 mM MgCl2, and 0.1 % (w/v) bovine serum albumin and 100 µg/ml bacitracin. Non-specific binding is determined as the binding in the presence of 1 µM of unlabeled PYY. Cells are washed twice in 0.5 ml of ice-cold buffer and 0.5-1 ml of lysis buffer (8 M Urea, 2 % NP40 in 3 M acetic acid) is added and the bound radioactivity is counted in a gamma counter. Determinations are made in duplicate. Steady state binding is reached with the radioactive ligand under these conditions. EC50 values were calculated using a standard pharmacological data handling software, Prism 3.0 (graphPad Sofware, San Diego, USA).

### Human Y4 receptor Affinity Assay

Protocol as for the Y2 affinity assay, except that human Y4-transformed COS-7 cells are used, the competition assay uses 125I-PP, and PP is used for the determination of non-specific binding.

### Human Y1 receptor Affinity Assay

Protocol as for the Y2 affinity assay, except that human Y1-transformed COS-7 cells are used and are transferred to culture plates at a density of 1.5 x 106 cells per well. the competition assay uses 125I-NPY, and NPY is used for the determination of non-specific binding.

### Human Y5 receptor Affinity Assay

Protocol as for the Y2 affinity assay, except that human Y5-transformed COS-7 cells are used and are transferred to culture plates at a density of 5 x 105 cells per well. the competition assay uses 125I-NPY, and NPY is used for the determination of non-specific binding.

The results of testing NPY, PYY. PYY3-36, PP and PP2-36 in the above affinity assays are given in Table 1:

**Table 1**

| Agonist | Y2 | | | Y1 | | | Y4 | | |
|---|---|---|---|---|---|---|---|---|---|
| | IC50 Nm | SEM | n | IC50 nm | SEM | n | IC50 nm | SEM | n |
| NPY | 0.30 | 0.07 | 4 | 2.3 | 0.3 | 4 | 26 | 5 | 4 |
| PYY | 0.22 | 0.02 | 2 | 16 | 1 | 2 | 30 | 8 | 2 |
| PYY(3-36) | 0.20 | 0.03 | 10 | >1000 | | 2 | 343 | 42 | 4 |
| PP | >1000 | | 4 | >1000 | | 1 | 0.41 | 0.05 | 11 |
| | | | | | | | | | |
| PP2-26 | >1000 | | 3 | >1000 | | 2 | 0.64 | 0.04 | 4 |

### Human Y2 receptor Potency Assay

Potency of the test compounds on the human Y2 receptor is determined by performing dose-response experiments in COS-7 cells transiently transfected with the human Y2 receptor as well as a promiscuous G protein, Gqi5 which ensures that the Y2 receptor couples through a Gq pathway leading to an increase in inositol phosphate turnover.

*Phosphatidylinositol turnover-* One day after transfection COS-7 cells are incubated for 24 hours with 5 µCi of [3H]-myo-inositol (Amersham, PT6-271) in 1 ml medium supplemented with 10% fetal calf serum, 2 mM glutamine and 0.01 mg/ml gentamicin per well. Cells are washed twice in buffer, 20 mM HEPES, pH 7.4, supplemented with 140 mM NaCl, 5 mM KCI, 1 mM MgSO4, 1 mM CaCl2, 10 mM glucose, 0.05 % (w/v) bovine serum; and are incubated in 0.5 ml buffer supplemented with 10 mM LiCl at 37C for 30 min. After stimulation with various concentrations of peptide for 45 min at 37C, cells are extracted with 10 % ice-cold perchloric acid followed by incubation on ice for 30 min. The resulting supernatants are neutralized with KOH in HEPES buffer, and the generated [3H]-inositol phosphate are purified on Bio-Rad AG 1-X8 anion-exchange resin and counted in a beta counter. Determinations are made in duplicates. EC50 values were calculated using a standard pharmacological data handling software, Prism 3.0 (graphPad Sofware, San Diego, USA).

### Human Y4 receptor Potency Assay

Protocol as for the Y2 potency assay, except that human Y4-transformed COS-7 cells are used.

### Human Y1 receptor Potency Assay

Protocol as for the Y2 potency assay, except that human Y1-transformed COS-7 cells are used.

### Human Y5 receptor Potency Assay

Protocol as for the Y2 potency assay, except that human Y5-transformed COS-7 cells are used

The results of testing NPY, PYY, PYY3-36, PP and PP2-36 in the above potency assays are given in Table 2:

**Table 2**

| Agonist | Y2 | | | Y1 | | | Y4 | | |
|---|---|---|---|---|---|---|---|---|---|
| | EC50 nm | SEM | n | EC50 nm | SEM | n | EC50 nm | SEM | n |
| NPY | 1.5 | 0.5 | 11 | 1.7 | 0.4 | 11 | 167 | 56 | 7 |
| PYY | 0.23 | 0.06 | 8 | 0.6 | 0.1 | 5 | 34 | 5 | 6 |
| PYY(3-36) | 0.36 | 0.07 | 16 | 74 | 5 | 7 | >1000 | | 8 |
| PP | >1000 | | 8 | 83 | 13 | 5 | 0.64 | 0.07 | 17 |
| | | | | | | | | | |
| PP 2-36 | >1000 | | 4 | 297 | 45 | 3 | 0.88 | 0.12 | 5 |

### II. IN VITRO ASSAYS TO DETERMINE PROTEIN STABILITY

An important measure for PP2-36 is the protein stability, especially in respect of stability for example towards degradation by enzymes as the peptide has been designed to have increased stability as compared to for example PYY3-36 or even increased stability as compared to full length PYY and PP.

**Stability of the PP-fold -** is determined as the stability of the peptide towards degradation by endopeptidases which cleave for example in the loop region, which is relatively flexible as described (O'Hare, M. & Schwartz, T.W. 1990 In Degradation of Bioactive Substances: Physiology and Pathophysiology. J.Henriksen, ed. CRC Press, Boca Raton, FI*.*). As a model enzyme, endoproteinase Asp-N (Pierce) is used. This enzyme cleave at the N-terminal side of Asp residues, for example between residues 9 and 10 (Asp) in PP. The peptide is incubated with an efficacious dose of endopeptidase Asp-N as indicated by the manufacturer in 0.01 M Tris/HCl buffer, pH 7.5 at room temperature and samples removed after various time periods over 24 hours. The samples are analysed by HPLC and the gradual degradation of the peptide is followed over time. The peptide is compared in respect of stability to PYY, PYY13-36 and PP.

**Stability towards aminopeptidases** - is determined as described above for endopeptidases but using aminopeptidase N and di-peptidylpeptidase IV in stead. The peptides are compared in respect of stability to PYY, PYY3-36 and PP.

### III. IN VIVO STUDIES TO DETERMINE THE EFFECT OF PP2-36 ON APPETITE, FOOD INTAKE AND BODY WEIGHT

### Effect of PP2-36 on acute food intake in mice

The ddy strain of mice were used, 34-37 g and 8-9 weeks of age (Japan SLC, Shizuuoka, Japan). The mice were individually house in a regulated environment (22 C, 55 % humidity) in a 12-hour light-dark cycle with light on at 7 AM. Food and water were available ad libitum except just before experiments (see below). The mice were acclimatized to subcutaneous injections during the week before experiments started. Mice were used once each in the experiments. Just before administration the peptides were diluted in physiological saline and administered in 100 µL volume for subcutaneous administration. Results are expressed as mean +/- SE. Analysis of variance followed by Bonferroni's test were used to assess differences among groups.

Mice were deprived of food but with free access to water for 16 hours prior to the actual test and experiments were started at 10 AM on the following day. A standard diet was used (CLEA Japan, Inc., Tokyo, Japan) and food intake was measured by subtracting uneaten food from the initially premeausured food after administration and checking for food spillage. Eight animals in each group receiving either saline, 1 µg PP2-36, or 10 µg PP2-36 test compound.

The results for PP2-36 (Seq ID No: 10) as test compound are shown in Fig. 2.

SEQUENCES:

NPY (SEQ ID No: 1)
H2N-Tyr--Pro-Ser-Lys-Pro-Asp-Asn-Pro-Gly-Glu-Asp-Ala-Pro-Ala-Glu-Asp-Met-Ala-
Arg-Tyr-Tyr-Ser-Ala-Leu-Arg-His-Tyr-Ile-Asn-Leu-Ile-Thr-Arg-Gln-Arg-Tyr-CONH2

PYY (SEQ ID No: 2)

H2N-Tyr-Pro-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Leu-Asn-Leu-Val-Thr-Arg-Gln-Arg-Tyr-CONH2

PP (SEQ ID NO: 3)

H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Ile-Asn-Met-Leu-Thr-Arg-Pro-Arg-Tyr-CONH2

[Cys2,DCys27]-PP SEQ ID. No:4)

S
|
H2N-Ala-Cys-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-(D-Cys)-Ile-Asn-Met-Leu-Thr-Arg-Pro-Arg-Tyr-
CONH2
|
S

PP2-36 (SEQ ID. No:10).

H2N-Pro-Leu-Glu-Pro-Val-Tyr-Pro-Gly-Asp-Asn-Ala-Thr-Pro-Glu-Gln-Met-Ala-Gln-
Tyr-Ala-Ala-Asp-Leu-Arg-Arg-Tyr-Ile-Asn-Met-Leu-Thr-Arg-Pro-Arg-Tyr-CONH2

## Claims

1. The use of a Y receptor agonist which is selective for the Y4 receptor over the Y1 and Y2 receptors, said Y receptor agonist being pancreatic peptide PP2-36 with SEQ ID No:10, in the preparation of a composition for (i) reducing food intake, (ii) treatment of obesity or overweight, (iii) treatment of diarrhoea or hypersecretion associated with small intestinal ileo-stomia or (iv) treatment of a condition in which obesity or overweight is considered a contributory factor, selected from bulimia, bulimia nervosa, Syndrome X (metabolic syndrome), diabetes, type 2 diabetes mellitus or Non Insulin Dependent Diabetes Mellitus (NIDDM), hyperglycemia, insulin resistance, impaired glucose tolerance, cardiovascular disease, hypertension, atherosclerosis, coronary artery disease, myocardial infarction, peripheral vascular disease, stroke, thromboembolic disease, hypercholesterolemia, hyperlipidemia, gallbladder disease, osteoarthritis, sleep apnea, polycystic ovarian syndrome, and cancer of the breast, prostate, or colon.

2. The use of pancreatic peptide PP2-36 with SEQ ID No:10, in the preparation of a composition for reducing food intake.

3. The use of a Y receptor agonist which is selective for the Y4 receptor over the Y1 and Y2 receptors, said Y receptor agonist being pancreatic peptide PP2-36 with SEQ ID No: -10, in the preparation of a composition for treatment of diarrhoea or hypersecretion associated with small intestinal ileo-stomia.

4. The use as claimed in any of claims 1 to 3, wherein the composition is adapted for administered to a patient via a subcutaneous, intramuscular, intravenous, nasal, transdermal or buccal route.

## Patentansprüche

1. Verwendung eines Y-Rezeptor-Agonisten, der für den Y4-Rezeptor gegenüber den Y1- und Y2-Rezeptoren selektiv ist, wobei der Y-Rezeptor-Agonist das pankreatische Peptid PP2-36 mit der SEQ ID NO: 10 ist, zur Herstellung einer Zusammensetzung (i) zum Reduzieren der Nahrungsaufnahme, (ii) zur Behandlung von Fettleibigkeit oder Übergewicht, (iii) zur Behandlung von Diarrhöe oder Hypersekretion, die mit Dünndarm-Ileostomie assoziiert ist, oder (iv) zur Behandlung eines Befunds, bei dem Fettleibigkeit oder Übergewicht als beitragender Faktor angesehen wird, ausgewählt aus Bulimie, Bulimia nervosa, Syndrom X (metabolisches Syndrom), Diabetes, Typ 2 Diabetes mellitus oder nicht-insulinabhängigem Diabetes mellitus (NIDDM), Hyperglykämie, Insulinresistenz, beeinträchtigter Glucosetoleranz, Herz-Kreislauf-Erkrankung, Bluthochdruck, Atherosklerose, Herzkranzarterienerkrankung, Myocardinfarkt, Erkrankung der peripheren Blutgefässe, Schlaganfall, thromboembolischer Erkrankung, Hypercholesterinämie, Hyperlipidämie, Gallenblasenerkrankung, Osteoarthritis, Schlafapnoe, polycystischem Eierstocksyndrom und Brust-, Prostata- oder Dickdarmkrebs.

2. Verwendung des pankreatischen Peptids PP2-36 mit der SEQ ID NO: 10 zur Herstellung einer Zusammensetzung zum Reduzieren der Nahrungsaufnahme.

3. Verwendung eines Y-Rezeptor-Agonisten, der für den Y4-Rezeptor gegenüber den Y1- und Y2-Rezeptoren selektiv ist, wobei der Y-Rezeptor-Agonist das pankreatische Peptid PP2-36 mit der SEQ ID NO: 10 ist, zur Herstellung einer Zusammensetzung zur Behandlung von Diarrhöe oder Hypersekretion, die mit Dünndarm-Ileostomie assoziiert ist

4. Verwendung wie in einem der Ansprüche 1 bis 3 beansprucht, wobei die Zusammensetzung für die Verabreichung an einen Patienten über den subkutanen, intramuskulären, intravenösen, nasalen, transdermalen oder bukkalen Weg angepasst wird.

## Revendications

1. Utilisation d'un agoniste du récepteur Y qui est sélectif vis-à-vis du récepteur Y4 par rapport aux récepteurs Y1 et Y2, ledit agoniste du récepteur Y étant le peptide pancréatique PP2-36 ayant la SEQ ID N°:10, dans la préparation d'une composition destinée (i) à la réduction de la prise alimentaire, (ii) au traitement de l'obésité ou de la surcharge pondérale, (iii) au traitement de la diarrhée ou de l'hypersécrétion associée à l'iléostomie de l'intestin grêle ou (iv) au traitement d'une affection dans laquelle l'obésité ou la surcharge pondérale est considérée comme facteur favorisant, choisie parmi la boulimie, la boulimie nerveuse, le syndrome X (syndrome métabolique), le diabète, le diabète sucré de type 2 ou le diabète sucré non insulinodépendant (NIDDM), l'hyperglycémie, la résistance à l'insuline, l'intolérance au glucose, les maladies cardiovasculaires, l'hypertension, fathérosclérose, la coronaropathie, l'infarctus du myocarde, les maladies vasculaires périphériques, l'accident vasculaire cérébral, la maladie thromboembolique, l'hypercholestérolémie, l'hyperlipidémie, la maladie de la vésicule biliaire, l'arthrose, l'apnée du sommeil, le syndrome des ovaires polykystiques et le cancer du sein, de la prostate ou du côlon.

2. Utilisation du peptide pancréatique PP2-36 ayant la SEQ ID N°:10 dans la préparation d'une composition destinée à la réduction de la prise alimentaire.

3. Utilisation d'un agoniste du récepteur Y qui est sélectif vis-à-vis du récepteur Y4 par rapport aux récepteurs Y1 et Y2, ledit agoniste du récepteur Y étant le peptide pancréatique PP2-36 ayant la SEQ ID N°:10, dans la préparation d'une composition destinée au traitement de la diarrhée ou de l'hypersécrétion associée à l'iléostomie de l'intestin grêle.

4. Utilisation telle que revendiquée dans l'une quelconque des revendications 1 à 3, où la composition est adaptée pour être administrée à un patient par voie sous-cutanée, intramusculaire, intraveineuse, nasale, transdermique ou buccale.
